(19) **Europäisches Patentamt**
**European Patent Office**
**Office européen des brevets**

(11) **EP 2 652 655 B1**

(12) # EUROPEAN PATENT SPECIFICATION

(45) Date of publication and mention
of the grant of the patent:
**15.05.2019 Bulletin 2019/20**

(51) Int Cl.:
***G06F 19/00*** *(2018.01)* ***A61K 31/122*** *(2006.01)*

(21) Application number: **11808568.7**

(86) International application number:
**PCT/DK2011/050462**

(22) Date of filing: **06.12.2011**

(87) International publication number:
**WO 2012/079576 (21.06.2012 Gazette 2012/25)**

(54) **METHODS AND MODELS FOR PREDICTING DOSAGE OF A DRUG AND/OR RESPONSE IN AN INDIVIDUAL**

VERFAHREN UND MODELLE ZUR VORHERSAGE DER DOSIERUNG EINES ARZNEIMITTELS UND/ODER REAKTION BEI EINER PERSON

MÉTHODES ET MODÈLES DE PRÉDICTION DU DOSAGE D'UN MÉDICAMENT ET/OU DE LA RÉPONSE CHEZ UN INDIVIDU

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.12.2010 DK 201001130**

(43) Date of publication of application:
**23.10.2013 Bulletin 2013/43**

(73) Proprietors:
• **Region Nordjylland**
**9220 Aalborg Øst (DK)**
• **Aalborg Universitet**
**9220 Aalborg Øst (DK)**

(72) Inventors:
• **LUNDBYE-CHRISTENSEN, Søren**
**DK-9000 Aalborg (DK)**
• **NIELSEN, Peter Brønnum**
**DK-9700 Brønderslev (DK)**
• **LARSEN, Torben Bjerregaard**
**DK-8240 Risskov (DK)**
• **HEJLESEN, Ole Kristian**
**DK-9800 Hjørring (DK)**

(74) Representative: **Plougmann Vingtoft a/s**
**Strandvejen 70**
**2900 Hellerup (DK)**

(56) References cited:
• Nielsen P. B.: "Modelling in Oral Anticoagulation Treatment", Ph.D. Thesis, 30 August 2010 (2010-08-30), pages 1-33, XP055002864, Aalborg University, Denmark ISBN: 978-8-77-094084-9 Retrieved from the Internet: URL:http://vbn.aau.dk/files/44591083/PhDThesis_PeterBroennumNielsen.pdf [retrieved on 2011-07-15]
• NIELSEN P. B. ET AL: "Assessing Importance of Dietary Data in Anticoagulation Treatment", STUD. HEALTH TECHNOL. INFORM., vol. 150, 30 August 2009 (2009-08-30), pages 782-786, XP055002896, DOI: 10.3233/978-1-60750-044-5-782 cited in the application
• PANNOCCHIA G. ET AL: "Model predictive control for optimal oral anticoagulant drug administration", AICHE JOURNAL, vol. 52, no. 9, 1 September 2006 (2006-09-01), pages 3315-3320, XP055002908, ISSN: 0001-1541, DOI: 10.1002/aic.10930 cited in the application
• VADHER B. ET AL: "Prediction of the international normalized ratio and maintenance dose during the initiation of warfarin therapy", BRITISH JOURNAL OF CLINICAL PHARMACOLOGY, vol. 48, no. 1, 1 July 1999 (1999-07-01), pages 63-70, XP055002840, ISSN: 0306-5251, DOI: 10.1046/j.1365-2125.1999.00967.x

**(Cont. next page)**

• ANSELL J. ET AL: "The Pharmacology and Management of the Vitamin K Antagonists: The Seventh ACCP Conference on Antithrombotic and Thrombolytic Therapy", CHEST, vol. 126, no. 3_suppl, 1 September 2004 (2004-09-01), pages 204S-233S, XP055002827, ISSN: 0012-3692, DOI: 10.1378/chest.126.3_suppl.204S

• SAWYER W. T. ET AL: "Digital computer-assisted warfarin therapy: Comparison of two models", COMPUTERS AND BIOMEDICAL RESEARCH, ACADEMIC PRESS, LONDON, GB, vol. 12, no. 3, 1 June 1979 (1979-06-01), pages 221-231, XP022956367, ISSN: 0010-4809, DOI: DOI:10.1016/0010-4809(79)90017-X [retrieved on 1979-06-01]

**Description**

**FIELD OF THE INVENTION**

The present invention relates to methods and devices for predicting dosage and/or response in an individual. More specifically, it relates to methods and devices for predicting dosage of an anticoagulant and/or the response to said anticoagulant in a patient receiving anticoagulant treatment.

**BACKGROUND OF THE INVENTION**

Patients with an increased risk of thrombosis require treatment with vitamin K-antagonists such as warfarin. Treatment with warfarin has been reported difficult mainly due to high inter- and intraindividual response to the drug. Several patients at risk of thrombosis will need some kind of anticoagulation therapy. Oral anticoagulation therapy (OAT) with coumarins (vitamin K-antagonists) is prescribed both for prophylaxis and therapeutically to a large group of patients at increased risk of thromboembolism, e.g. patients with atrial fibrillation, heart valve replacement, deep venous thrombosis, and pulmonary embolism.[1]

The most serious adverse effect of OAT is bleeding. The treatment attempts to balance between avoiding haemorrhages due to over-treatment and recurrence of thrombotic events due to insufficient OAT. The quality of the treatment is usually assessed by measuring the International Normalized Ratio (INR) value. This ratio represents a patient's coagulation time (prothrombin time) compared to a normal individual. Maintaining patients within the desired therapeutic window of INR values, which is between 2.0 and 3.0 for most patients, presents a challenge due to at least three factors: (1) a target INR value restricted by a relatively narrow therapeutic range, (2) an inter-individual variation of the effect of oral vitamin K antagonists, and (3) changes in dietary intake of vitamin K.[2, 3] In other words, efficacy and safety of OAT are dependent on the maintenance of the INR in a narrow range recommended by current practice guidelines.[4] Despite tight control, the time in therapeutic range has in large studies been reported to be below 70% and INR range-dependent.[5, 6] The fact that no simple relationship exists between a vitamin K-antagonist (VKA) dose and the therapeutic effect might be an explanatory factor for relatively low adherence to INR in therapeutic range. The pharmacological characteristics of anticoagulant agents are in general well documented, while the management of warfarin is still a complex task. Warfarin is difficult to dose correctly. This is mainly due to inter- and intraindividual variability between patients' response to the required maintenance dose. [7]

OAT is predominantly monitored by laboratory determination of INR using plasma obtained by venipuncture. Another method is patient self-management (PSM), in which the patients analyze a drop of blood using a portable coagulometer (INR-monitor) and subsequently determine their dose of OAT independently. At present, PSM has shown good clinical results in selected patients, and these patients will typically measure INR once a week.[8]

Some patients assigned to PSM are using decision aiding electronic tools, an online decision support system that will provide an on-screen dosage advice. Such tools have been described and refined in the literature over the past 30 years. [9, 10] Common for these systems are an attempt of interpretation of prothrombin time (often provided as INR), to provide an advice on optimal VKA dose, and for some systems to give an estimate of when next measurement/test is needed. Their applications are mainly utilized in warfarin initiation or in the warfarin maintenance phase (or both). Other areas of possible use of such systems could be aid in achieving a new INR target in a post-operative situation.

In general, model based approaches in a broad field of medical treatment and monitoring has been described. Harris suggested a class of auto regressive models for within individual variation in blood constituents. [11, 12] Similar models have been used for monitoring tumour markers in small cell lung cancer and breast cancer.[13-15] State space modelling techniques have been applied in monitoring of medical parameters that develops over time, where one of the first examples being the monitoring of renal transplants by Smith and West who used a multi process Kalman filter for change point detection.[16] Alternatively Cusum techniques have been suggested for detecting changes in the behaviour of biomarker series.[17] A general auto regressive predictive model for glucose levels in diabetic patients is applied, and provided sufficiently accurate estimates of glucose levels.[18] An attempt using a state-space model to provide warfarin dose advices has been proposed by Pannocchia and Brambilla.[19] This approach handles the initial state and noise estimation from patient data, and the algorithm attempts to keep the INR value close to the target INR or within the desired therapeutic range.

**SUMMARY OF THE INVENTION**

In a first aspect, the present invention relates to a method according to the appended claims.

In a second aspect, the present invention relates to a dosage-response calculator according to the appended claims.

As an example, there is presented a dynamic prediction model for predicting dosage and/or response in an individual, said dynamic prediction model comprising equation (i):

$$R_t = T + \sum_{i=1}^{k} a_i(d_{t-i} - D) + \varepsilon_t + u_t \qquad (i);$$

wherein "$R_t$" is the response at time "t", "T" is the target response, "a" is the individual sensitivity towards dosage variation, "$d_t$" is the administered dose at time "t", "D" is the theoretical dose to administer for obtaining "T", "$\varepsilon_t$" is a autoregressive process, and "$u_t$" is white noise characterized by being mutually independent, normally distributed with parameters N(0, $T^2$).

## DESCRIPTION OF THE FIGURES

Figure 1: A representation of Equation 18 based on values of $\lambda$ and $\rho$ ranging from [0;1]. The areas with most bright colours depict large values of the log-likelihood function.

Figure 2: Retrospectively model based INR predictions (solid line marked with squares) on patient data, and measured INR values for the patient (solid line marked with circles). Bars represents warfarin intake scaled 1:5mg (=2 tablets), and dashed line shows the theoretical correct warfarin dose (D in Equation 6) to keep the patient on INR target = 2.5.

Figure 3: The autocorrelation plot from lag 0 to 25 on residual error produced from model predictions. The horizontal dashed lines indicate approximate 95% confidence limits.

Figure 4: Simulated warfarin data where INR measurements (solid line marked with circles) are not provided from day 22 on forward. Bars represents warfarin intake scaled 1:5mg (=2 tablets).

Figure 5: An illustration of performance on simulated warfarin (bars) data and simulated INR data (solid line marked with circles).Warfarin intake is halted at day 28 and doubled at day 40. Bars represents warfarin intake scaled 1:5mg (=2 tablets), and dashed line shows the theoretical correct warfarin dose to keep the INR at target 2.5.

## DETAILED DESCRIPTION OF THE INVENTION

The present invention relates to general methods for prediction of dosage and/or response in an individual. In addition, as examples there are presented general models for predicting dosage and/or response in an individual. Further, the present invention relates to a general dosage-response calculator being able to calculate the dosage and/or response in an individual. It is within the scope of the present invention, that the methods and dosage-response calculators presented herein, all are suitable for predicting the dosage and/or response in an individual suffering from any kind of disease/condition. However, as presented herein below, the methods and dosage-response calculators of the invention are particular useful for predicting dosage and/or response in an individual receiving anticoagulant treatment.

### Methods, models and dosage-response calculators for predicting dosage and/or response in an individual

In one aspect, the present invention relates to a method according to the appended claims.

In another aspect, the present invention relates to a dosage-response calculator according to the appended claims.

As an example there is presented a dynamic prediction model for predicting dosage and/or response in an individual, said dynamic prediction model comprising equation (i):

$$R_t = T + \sum_{i=1}^{k} a_i(d_{t-i} - D) + \varepsilon_t + u_t \qquad (i);$$

wherein "Rt" is the response at time "t", "T" is the target response, "a" is the individual sensitivity towards dosage variation, "$d_t$" is the administered dose at time "t", "D" is the theoretical dose to administer for obtaining "T", "$\varepsilon_t$" is a autoregressive

process, and "$u_t$" is white noise characterized by being mutually independent, normally distributed with parameters N(0, $T^2$).

It is an object of the present invention, that the result obtained from above method in step c) and/or from the above dosage-response calculator in step c), may be used to predict the dosage and/or response in an individual receiving treatment for a disease/condition.

In one embodiment of the present invention, the individual is a patient receiving anticoagulant treatment, and hence the method and dosage-response calculator for predicting dosage and/or response in said patient, may be specified to utilize a simplified version of above equation (i), namely equation (ii), which is identical to equation (3) also presented herein further below:

$$INR_t = T + a_1(d_{t-1} - D) + a_2(d_{t-2} - D) + \varepsilon_t \qquad \text{(ii) or (3)};$$

wherein $INR_t$ is the International Normalized Ratio to time "t". In the above case, where the individual is a patient receiving anticoagulant treatment, the autoregressive process is a first-order autoregressive process (AR(1)), and may be expressed by equation (iii), which is identical to equation (2) also presented herein further below:

$$\varepsilon_t = \rho \cdot \varepsilon_{t-1} + \nu_t \qquad \text{(iii) or (2)};$$

wherein "$\rho$" is a constant quantifying the day-to-day correlation, and "$\nu_t$" are mutually independent, normally distributed with parameters N(0, $\sigma^2$).

It is within the scope of the present invention, "$\nu_t$" and "ut" are mutually independent, as well as independent of each other.

It is also within the scope of the present invention, that the methods and dosage-response calculators presented herein, may be further refined by applying state-space modeling and Kalman filtering, as described herein below in Example 1.

**Variables**

The one or more variables used in the methods and dosage-response calculators of the present invention, may be any variable comprised within any equation presented herein, for example above equation (i) and equation (ii), identical to equation (3) presented herein further below, such as "$R_t$" and/or "$INR_t$" and/or "T" and/or "a" and/or "$d_t$" and/or "D" and/or "$\varepsilon_t$" and/or "$u_t$". It is within the scope of the present invention, that any variable described herein may be used as input variable in the methods and dosage-response calculators of the invention. It is also within the scope of the present invention, that any variable described herein may serve as output variable, or result, in the methods and dosage-response calculators of the invention.

In a certain embodiment of the present invention, the variables "T", and/or "$d_t$" and/or "$R_t$", such as "$INR_t$", are known or observable/measureable variables, and thus are used as input variables in the methods and dosage-response calculators of the invention, while the variables "a" and/or "D" are unknown or latent variables, and thus serve as output variables in the methods and dosage-response calculators of the invention.

It is within the scope of the present invention, that the time variable "t" may refer to any unit of time, such as microseconds, milliseconds, seconds, minutes, hours, days, weeks, months or even years. I one embodiment of the present invention, the time variable "t" refers to minutes, hours, days or weeks. In a preferred embodiment of the present invention, the time variable "t" refers to days.

It is also within the scope of the present invention, that sigma-function of above described equation (i), may sum from i=1 to k, wherein k may be any positive integer. In a certain embodiment of the present invention, the positive integer k equals 2, in such way that above equation (i) may be simplified to above equation (ii), identical to equation (3) presented herein further below.

As stated above, the variable "$INR_t$" corresponds to the International Normalized Ratio (INR) to time "t". The INR is a derived from the prothrombin time (PT), a measure of the extrinsic pathway of blood coagulation well known the skilled person in the art. During measurement of the prothrombin time, the enzyme "tissue factor" is added, and in order to standardize the results, each manufacturer of tissue factor must assign an ISI value (International Sensitivity Index) for each batch of tissue factor they produce. Hence the International Normalized Ration (INR) is defined by the ratio of the prothrombin time for a patient ($PT_{patient}$) to the prothrombin time for a control person ($PT_{control}$), raised to the power of the International Sensitivity Index for the enzymatic system used:

$$INR = (PT_{patent} / PT_{control})^{ISI}$$

As stated above, the variable "T" is the target response, i.e. the desired response "R". In a certain embodiment of the present invention, where the individual is a patient receiving anticoagulant treatment, the variable "T" is the target/desired "INR" value.

As also stated above, the variable "$d_t$" is the administered dose of a relevant drug to the individual at time "t". In a certain embodiment of the present invention, where the individual is a patient receiving anticoagulant treatment, the variable "$d_t$" is the dose of an anticoagulant, such as a vitamin K antagonist, to time "t". In a preferred embodiment of the present invention, where the individual is a patient receiving anticoagulant treatment, the variable "$d_t$" is the dose of warfarin.

As stated above, the variable "D" is the theoretical dose of a relevant drug to administer to an individual, in order to obtain the target response "T". In a certain embodiment of the present invention, where the individual is a patient receiving anticoagulant treatment, the variable "D" is the theoretical dose of an anticoagulant, such as a vitamin K antagonist, to administer to the patient in order to obtain target response "T", i.e. the target/desired "INR" value. In a preferred embodiment of the present invention, where the individual is a patient receiving anticoagulant treatment, the variable "D" is the theoretical dose of warfarin, to administer to the patient in order to obtain target response "T", i.e. the target/desired "INR" value.

As also stated above, the variable "a" is the individual sensitivity towards dosage variation of a relevant drug administered to the individual. In a certain embodiment of the present invention, where the individual is a patient receiving anticoagulant treatment, the variable "a" is the individual sensitivity towards dosage variation of an anticoagulant, such as a vitamin K antagonist administered to the patient. In a preferred embodiment of the present invention, where the individual is a patient receiving anticoagulant treatment, the variable "a" is the individual sensitivity towards dosage variation of warfarin administered to the patient.

**Dosage**

It is within the scope of the present invention, that dosage of any relevant drug administered to an individual according to the methods and dosage-response calculators of the invention, and hence the value of any variable relating to dosage presented herein, may be of any magnitude. It is also within the scope of the present invention, that said dosage of any relevant drug administered to an individual according to the methods and dosage-response calculators of the invention, and hence the value of any variable relating to dosage presented herein, may be specified in different ways known to the person skilled in the art, for example as

- amount, such as "g", "mg", "$\mu$g", "ng", "U" and "ppm"

- amount per time, such as "g/month", "mg/week", "$\mu$g/day", "ng/minute" or any relevant ration of amount per timeframe

- amount per weight, such as "g/kg", "mg/kg", "$\mu$g/kg", "ng/g" or any relevant ratio of amount per weight

- amount per volume, such as "g/l", "mg/l", "mg/ml", "$\mu$g/ml", "ng/$\mu$l" or any relevant ratio of amount per volume

**Medication monitoring in different diseases/conditions**

As stated elsewhere herein, the methods and dosage-response calculators of the present invention, are particular useful for predicting dosage and/or response in a patient receiving anticoagulant treatment. However, as also stated elsewhere herein, the methods and dosage-response calculators of the present invention may also be useful for predicting dosage and/or response in other diseases/conditions. Within the meaning of the present invention, "predicting dosage and/or response" is equivalent to "medication monitoring". Medication monitoring measures the serum level of a specific medicine to ensure a correct dosage. This is especially important when the drug has a narrow therapeutic index (effective dose is close to the toxic level). This index varies from person to person, and is affect of liver and kidney function, gender, age, diet etc. In patients with an altered clearance or half-life the medicine can become toxic.

Medication monitoring facilitated by the methods and dosage-response calculators of the present invention are relevant in, but not limited to, the below situations:

- Confirming an effective dose of medicine

- Determine the correct dose of medicine

- Determine the clearance of a drug or poison

- Ensure good compliance

Below are non-limiting examples of medicines, for which medication monitoring can be facilitated by the methods and dosage-response calculators of the present invention:

- Certain antibiotics (amikacin, gentamicin , tobramycin)

- Medicines used i cardiovascular medicine (digoxin, quinidine, procainamide)

- Medicines to treat epilepsy (phenytoin, carbamazepine, phenobarbital, valproic acid)

- Asthma medicine (theophylline).

- Medicines to treat bipolar disorder (lithium)

- High doses of aspirin or similar (salicylates, NSAID).

- Medicines used in organ transplantation (cyclosporine, tacrolimus, FK-506)

Below are non-limiting examples of the effects relating to different diseases/conditions, for which medication monitoring can be facilitated by the methods and dosage-response calculators of the present invention:

- Hypertension: Blood pressure

- Kidney function: Creatinine clearance (CCr)

- Pulmonary function: Forced expired volume in the first second (FEV1), peak expiratory flow (PEF), Oxygen saturation (SvO2)

- Chronic obstructive pulmonary disease (COPD): FEV1, PEF

- Blood acidity: pH

- Diabetes: Blood glucose, Hemoglobin A1C

- Monitoring infectious disease: C-reactive protein (CRP), erythrocyte sedimentation rate (ESR).

- Monitoring inflammatory disease: Interleukins IL-1, IL-6 and IL-8, CRP, ESR and TNF-$\alpha$.

- HIV monitoring: T-cell count test (CD4), Viral load tests, HIV drug resistance test

- Viral hepatitis monitoring: Monitoring of CBC (neutropenia and thrombocytopenia), hemoglobin (hemolytic anemia)

**Anticoagulants**

According to a certain embodiment of the present invention, the methods and dosage-response calculators of the invention, is particularly useful for predicting the dosage and/or response in a patient receiving anticoagulant treatment. It is within the scope of the present invention that the treatment may comprise administering to the patient, any anticoagulant known to the skilled person in the art, such as any substance interfering with the intrinsic- and/or extrinsic pathway of blood coagulation. Such anticoagulant may be any type of pharmaceutical vitamin K antagonist, such as any compound belonging the group of coumarins - in particular 4-Hydroxycoumarins - such as acenocoumarol and/or dicumarol and/or ethyl biscoumacetate and/or phenprocoumon and/or warfarin. But also potent vitamin K antagonists with prolonged time of action, such as coumatetralyl, difenacoum, flocoumafen, bromadiolone, coumatetralyl, tioclomarol and/or brodifacoum, may be within the prediction scope of the methods and dosage-response calculators of the present invention. In a preferred embodiment of the present invention, where the individual is a patient receiving anticoagulant treatment, the anticoagulant is any type of courmarin or coumarin derivative. In an especially preferred embodiment of the present invention, where the individual is a patient receiving anticoagulant treatment, the anticoagulant is warfarin.

**Calculator and devices**

The dosage-response calculator according to the present invention, may be integrated into any device or devices capable of performing calculations, based on one or more equations presented herein, using one or more variables presented herein as input, and being able to output and display one or more results. Thus, the dosage-response calculator of the present invention may be integrated to any device or devices, capable of predicting the dosage and/or response in an individual, comprising an input module for inputting one or more variables, a computation module for performing one or more calculations, and an output module for displaying one or more results. Such devices include, but are not limited to, computers, portable computers, labtops, NetBooks, PDA's, cellular phones, mobile phones, Smart-Phones, Tablets and Tablet computers, and any type of handheld computing device, such as a coagulameter, including CoaguCheck® devices. In one embodiment of the present invention, the calculator of the present invention is integrated directly into the above device or devices, and the user of the device(s) enter variables directly on the device, where after the result of the calculation is displayed on the device(s).

However, in another embodiment of the present invention, the calculator may be integrated into larger computing devices, such as servers and server parks comprising means and systems for handling data, such as databases, wherein the user remotely inputs one or more variables of the present invention as described above. This may be accomplished by utilizing any type of input interface, such as, but not limited to, input interfaces comprising public and/or private domain internet websites, intranet websites, systems accessible by VPN tunneling or any other type of system accessible by any type of secure or unsecure connectivity. After performing the appropriate calculations according to the calculator of the present invention, the result(s) are presented to the user utilizing any kind of output interface known to the person skilled in the art.

**Example 1:**

**Methods, models and dosage-response calculators in anticoagulant treatment**

The present invention relates in one embodiment to a dynamic predictive method and dosage-response calculator based on a state-space modelling approach. The algorithm will provide an individual sensitivity parameter to account for inter- and intraindividual response to warfarin, which may prove pivotal in clinical situations for long-term OAT. It takes warfarin intake and International Normalized Ratio (INR) values as input, and uses an individual sensitivity parameter to model response to warfarin intake. The model is set on state-space form and uses Kalman filtering technique to optimize individual parameters. Retrospective test of the model proved robustness to choices of initial parameters, and feasible prediction results of both INR values and suggested warfarin dosage.

All patients included in the development of the present invetion referred to Medical Outpatient Clinic, Braedstrup Hospital. Approval from the Danish Data Protection Agency was obtained prior to onset of the data acquisition program. Clinicians at the facility enrolled suitable patients to be included in a data collection protocol, and the study was conducted in the period from January 2007 till February 2009. Patients were asked to fill out a daily scheme for a one month period. Schemes included information (among others) on INR value, diet, and warfarin intake.

Dosage-response calculator

The dosage-response calculator will at a given time, t, require having obtained the following input from the user: a target T, past and current INR value ($INR_1$, ..., $INR_t$) values, and past doses ($d_1$, ..., $d_{t-1}$). Several of the INR values may be unobserved (not measured or not available). All doses are required.

Initially the calculator will require established population values of $m_0$, $C_0$, $s_0$ and no, where the variables quantifies

• the average theoretical correct dose ($m_{0,1}$)

• the average Warfarin sensitivity ($m_{0,2}$)

• the average deviation from target INR ($m_{0,3}$)

• the population variation of each value mentioned above expressed as variances and covariances. This variance/covariance matrix will be denoted $C_0$

- parameters for describing the distribution between patients in uncertainty in the error term $v_t$ ($s_0$ and $n_0$)

At time t the calculator will output a prediction of the following variables:

- Theoretical correct dose (D) to obtain target T

- Warfarin sensitivity value (a)

- INR-T at time t+1 ($\mu$)

- Uncertainties of each value mentioned above expressed as variances and covariances

- Updated parameter values according to patient specific s and n

In the following the predictions will be denoted m=[$m_1$, $m_2$, $m_3$] and the variance/covariance matrix will be denoted C.

The calculator combine at time t, the values of $INR_t$, $d_{t-1}$ and $d_{t-2}$ with $m_{t-1}$, $C_{t-1}$, $n_{t-1}$ and $s_{t-1}$ to obtain $m_t$, $C_t$, $n_t$ and $s_t$, according to below equations (11) - (16) and (16*)

Initial data analysis

A retrospective statistical evaluation of variability in INR values was performed. This initial data mining has the purpose of revealing relations between INR values and past actions affecting INR values. The current INR value is predicted from past INR values in a regression model, and revealed a simple auto-regressive model

$$INR_t = T + \varepsilon_t, \qquad (1)$$

where T is the patent individual target and $\varepsilon_t$ is a first order autoregressive process (AR(1)),

$$\varepsilon_t = \rho \cdot \varepsilon_{t-1} + v_t \qquad (2) \text{ or (iii)}$$

where $v_t \sim N(0, \sigma^2)$ is assumed, and the where the individual $v_t$ are assumed mutually independent. This model describes departures from desired INR target level, where $v_t$ is depicting biological variation, and p is a constant, which quantifies the day-to-day correlation. The warfarin intake is assumed in accordance with intent of maintaining INR values at that target level. The error term $\varepsilon_t$ describes all variations related to warfarin intake and other unexplained variation. An AR(1) model suffices to describe the variation (P = 0.937, against AR(4)). The autoregressive coefficients do not vary significantly between patients (P = 0. 190), but the standard deviation varies in the population (P = 0.016, by Levene's test). By introducing past warfarin intake values, we arrive at the following model

$$INR_t = T + a_1(d_{t-1} - D) + a_2(d_{t-2} - D) + \varepsilon_t \qquad (3) \text{ or (ii)}$$

where D is the patient specific dose corresponding to maintaining a desired target INR level, $a_1$ and $a_2$ describes the sensitivity to deviations from this dose, $d_t$, is the dose/intake of warfarin at day t. The error term $\varepsilon_t$ now describes unexplained variation from e.g. changes in dietary vitamin K, pharmacogenetic variations between patients, and possible other medication intake interfering with the OAT. The warfarin sensitivities ($a_1$ and $a_2$) proved significant between-patient variations (P = 0.0001) as in accordance with [7] and [20]. For simplicity the individual dosage, D, is assumed to be constant over time. Individual average dosage during the study period was used as an estimate of D. As in the model without warfarin intake (Eq. (1)), a first order AR(1) with p common to all patients proved to describe residual variation sufficiently (P = 0.690, against individual p and lag 4 dependence of warfarin). Residuals for this regression model were mutually uncorrelated and showed no deviations from normality.

Model development

The model from the described initial data analysis will be applied for INR value predictions. Established population coefficients will be utilized as initiation values and gradually be adopted into individual parameters. Hence each population parameter will gradually develop into patient specific parameters and each patient will be his or hers own reference. The sensitivities are expressed as a between day profile common to all patients, $\lambda$, and a single sensitivity factor, $A_t$, varying between patients, see Eq. (4).

$$a_1 = A_t \, , \, a_2 = A_t \cdot \lambda \qquad (4)$$

The ratio, $\lambda$, between the two sensitivities corresponds to weighting effects of intake of warfarin at day t-1 and day t-2. If $\lambda < 1$, the warfarin effect on INR at day t is mainly influenced by the intake at day $t_{-1}$.

To facilitate consecutive updating of parameters and making predictions, the model is put on state-space form as follows: Let $y_{p,t}$ for $t=1,...,n_p$ and $p=1,... P$ be the INR measurement at day t for the p'th patient. Indexing for patient is suppressed in the following for notational convenience.

$$y_t = \mu_t + T \qquad (5)$$

where $\mu_t$ is the deviation from target INR. Note that in contrast to usual state space models no measurement noise is modelled. The latent variables driving the process are dose, $D_t$, sensitivity, $A_t$, and deviations from target, $\mu_t$.

The evolution over time in deviation from target can be modelled as in Eq. (6).

$$\mu_t = \rho \, \mu_{t-1} + A_{t-1}(d_{t-1} - D_{t-1} + \lambda(d_{t-2} - D_{t-1})) + v_t. \qquad (6)$$

The sensitivity and likewise the individually recommended dose vary between patients but not over time. In order to fit the variables in state-space form, the time constancy is modelled as in Eq. (7).

$$D_t = D_{t-1} \text{ and } A_t = A_{t-1} \qquad (7)$$

If the model should allow for a dosage varying over time, this could be achieved by modelling the time evolution $D_t = D_{t-1}$ + noise. The standard deviation of the noise term reflects the expected magnitude of day-to-day changes in dosage.

The error terms ($v_t$) of Eq. (6) are assumed to be mutually independent, normally distributed with zero mean and a variance specific to the patient. By using an inverse gamma distribution for describing the between patient distribution, we can exploit the conjugate form when using the model for prediction. Hence

$$v_t \sim N(0, \phi^{-1}), \text{ and } \phi \sim Ga(n_0/2, s_0/2) \qquad (8)$$

Organizing the latent variables in a three dimensional vector:

$$\theta = \begin{bmatrix} D_t \\ A_t \\ \mu_t \end{bmatrix}$$

the evolution over time is $\theta_t = G(\theta_{t-1})$, where the evolution function is described in equations (6) and (7).

The dynamic process is commenced by specifying the distribution of $\theta_0$ as in Eq. (9).

$$\theta_0 \sim N(m_0, \phi^{-1} \cdot C_0) \qquad (9)$$

where $m_0 = (D_0, A_0, \mu_0)$ describes the population average dose, sensitivity, and deviation from target, and where $C_0$ quantifies the corresponding variance over the population. The parameters n0 and s0 quantify the variation between patients in individual variations as explained in Eq. (8). The diagonal matrix $C_0$ is defined such that $C_0 \cdot (s_0/n_0)$ is the population variance matrix of (individual) dose, sensitivity, and deviation from target INR. Note that by definition $C_{0,33} = 1$.

This model is a conjugate normal-gamma state-space model apart from the fact that the time evolution of $\theta_t$ is non-linear in $\mu_t$ (see Eq. (6)). Hence an approximate approach for prediction will be taken. Further, the measurement equation is specified without a variance component, as found in most state-space models.

The parameters $\rho$ and $\lambda$ describes the dynamics of the INR-warfarin interaction, whereas $m_0$, $C_0$, $n_0$ and so describes the distribution between patients.

Model predictions

By using the principles of Kalman filtering, the distribution of $\theta_t$ and $y_t$ given prior observations, $O_t = \{y_1, ..., y_{t-1}\}$, and $\phi$ will be approximated by a normal distribution with mean and variance given from the following Eq. (10-16). The process is initiated in Eq. (9) with values obtained in the initial data analysis.

Assume at time t - 1 the distribution of the latent process follows Eq. (10).

$$\theta_{t-1}|O_{t-1}, \phi \sim N(m_{t-1}, \phi^{-1}C_{t-1}),$$

$$\phi|O_{t-1} \sim Ga(n_{t-1}, s_{t-1}) \qquad (10)$$

This implies the conditional mean, at, and variance, $R_t$, of $\theta_t$ will be as in Eq. (11) and (12).

$$a_{t1} = m_{t-1,1}$$

$$a_{t2} = m_{t-1,2}$$

$$a_{t3} = \rho \cdot m_{t-1,3} + m_{t-1,2}(d_{t-1}+\lambda d_{t-2} - (1+\lambda) m_{t-1,1}) -(1+\lambda)C_{t-1,12}s_{t-1}/n_{t-1} \qquad (11)$$

and

$$R_t = B \, C_{t-1} \, B^T + H \qquad (12)$$

where H is a 3x3 matrix with $H_{ij}=0$ except for

$$H_{33} = 1 + (1+\lambda)^2 (C_{t-1,11}C_{t-1,22} + C^2_{t-1,12}) \, s_{t-1}/n_{t-1}$$

and

$$B_t = \begin{bmatrix} -(1+\lambda)a_{t-1,2} \\ d_{t-1} + \lambda d_{t-2} - (1+\lambda)a_{t-1,1} \\ \rho \end{bmatrix}$$

The conditional distribution is approximated with a normal distribution as in Eq. (13).

$$\theta_t|O_{t-1}, \phi \sim N(a_t ; \phi^{-1}R_t) \qquad (13)$$

and the predictive distribution of the theoretical INR follows Eq. (14).

$$y_t|O_{t-1}, \phi \sim N(f_t ; \phi^{-1}Q_t) \qquad (14)$$

with $f_t = a_{t,3} + T = F^T\theta_t$, where

$$F = \begin{bmatrix} 0 \\ 0 \\ 1 \end{bmatrix}$$

and $Q_t = R_{t,33}$.

Updating the distribution of $\theta_t$ for the new observation is done by conditioning with $y_t$ in the joint distribution of $(\theta_t, y_t)$;

$$\theta_t | O_t, \phi \sim N(m_t, \phi^{-1}C_t),$$

where

$$m_t = a_t + \frac{FR_t(y_t - f_t)}{Q_t} \qquad (15)$$

and

$$C_t = R_t - \frac{FR_t(F\,R_t)^T}{Q_t} \qquad (16)$$

The distribution of the precision of f is updated by

$$n_t = n_{t\text{-}1} + 1 \text{ and } s_t = s_{t\text{-}1} + (y_t - f_t)^2/Q_t. \qquad (16^*)$$

Note that $n_t = n_0 + t$, independently of data, whereas st measures the cumulated squared, normalized prediction error. In case of missing INR values or if long-term predictions are required, the distribution of $\theta_t$ is not updated as in Eq. (15) and (16), but rather mt = at and $C_t = R_t$. The precision parameters are left unchanged, $n_t = n_{t\text{-}1}$ and $s_t = s_{t\text{-}1}$. The recursive characteristic of the algorithm is hereby maintained.

Parameter optimization

The parameters used in the predictive model can be divided into population parameters and dynamic parameters. As the model predictions require initial values for population parameters, moment estimates on $m_0$, $C_0$, $n_0$, and $s_0$, based on the initial data analysis, are used. Further, the dynamic parameters $\lambda$ and $\rho$ are estimated by a log-likelihood optimization, where values for both parameters are iteratively adjusted. The likelihood function is given as the product of the predictive densities obtained from Eq. (14). By marginalizing out $\phi$ the predictive distribution will be a T-distribution. Hence the likelihood function is as shown in Eq. (17).

$$L(\rho, \lambda) \propto \prod_p \prod_t \left(1 + \frac{e_{p,t}^2}{n_{t-1}}\right)^{\frac{-n_{t-1}+1}{2}} \qquad (17)$$

where the prediction error normalized by individual standard deviation, $e_{pt}$, is modelled as in Eq. (18).

$$e_{pt} = \frac{y_{p,t} - f_{p,t}}{\sqrt{Q_{p,t}\frac{s_{p,t-1}}{n_{t-1}}}} \qquad (18)$$

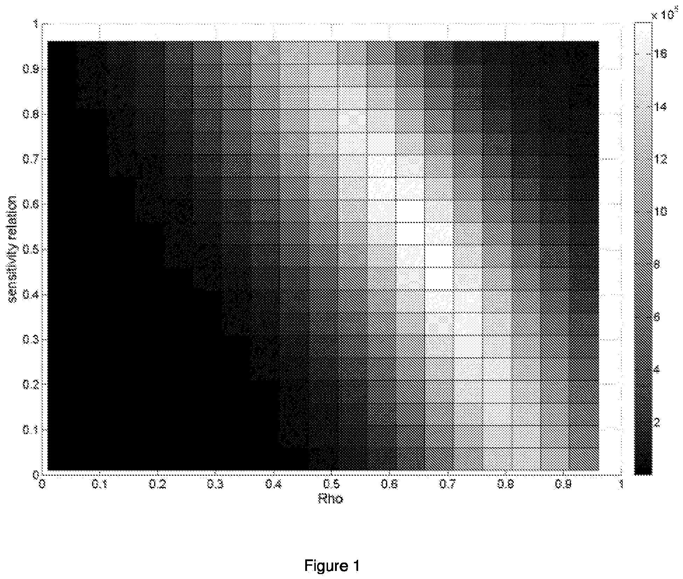

where $p$ is patient index and $t$ is the observation number. A graphical representation of the log-likelihood function for $\lambda$ and $\rho$ is given in Figure 1.

By maximizing the likelihood function for $\lambda$ and $\rho$, we derive the following optimal values $\rho$=0.61 and $\lambda$=0.56.

Results

Thirty patients accepted to be contacted, six declined to participate. Out of the 24 patients going into the study, 18 patients completed the data collection protocol (Table 1), equivalent to a 25% dropout rate.

*Table 1: Outline of 18 patients receiving warfarin. (Abbreviations: Std. Deviation$_{between}$ and Std. Deviation$_{within}$)*

| Characteristics | Number (%) or Mean $\pm$ SD$_b$, SD$_w$ |
|---|---|
| Age (years) | 56 $\pm$ 15 |
| Females | 8 (44%) |
| Days in study | 27 $\pm$ 1.78 |
| INR value | 2.5 $\pm$ 0.36, 0.37 |
| Warfarin intake [mg/day] | 6 $\pm$ 0.92, 0.43 |

Model performance

Two error terms are selected to evaluate the model performance: ept from Eq. (17) is selected as this error term describes predictive error from the model, but accounts for expected between patient variations. Another error term to be evaluated is chosen to be the Root Mean Square error (RMS), given as in Eq. (19).

$$RMS_p = \sqrt{\frac{1}{n_p}\sum_t (y_{p,t} - f_{p,t})^2} \qquad (19)$$

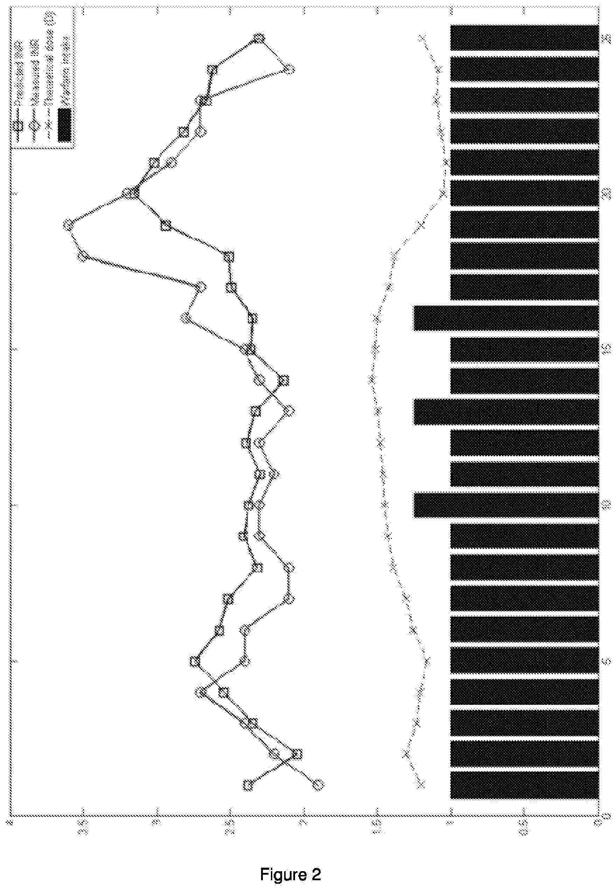

The RMS error is a non-weighted error that do not account for expected individual variance. The model was used to produce predictions on INR values for all 18 patients; and example for a single patient is given in Fig. 2.

Model control

To assess the influence from initial population values obtained from the initial data analysis, a sensitivity analysis is performed on the model. As mentioned in comments to Eq. (7), the modelled warfarin intake (theoretical correct dose) could be regarded as dynamic. This corresponds to allowing a warfarin dose to change over a week with e.g. 2.5mg/week (one tablet). The sensitivity analysis towards initial values is performed by adjusting values for parameters seen in Table 2.

*Table 2: Summary of the sensitivity analysis concerning initial values.*

| | | Static dose | | Dynamic dose | |
|---|---|---|---|---|---|
| Mean parameters | Adjustment | $\sum$RMS 0.4049 | $\sum e^2_{pt}$ 1.2214 | $\sum$RMS 0.2663 | $\sum e^2_{pt}$ 1.0563 |
| Dose $m_{0,1}$= 6.022 | ½· $m_{0,1}$ | 1.2626 | 1.4086 | 0.3999 | 1.0917 |
| | 2·$m_{0,1}$ | 1.9642 | 1.7011 | 0.5800 | 1.1059 |
| Sensitivity $m_{0,2}$= 0.079 | ½· $m_{0,2}$ | 0.4991 | 1.0433 | 0.2582 | 1.0568 |
| | 2·$m_{0,2}$ | 0.5363 | 1.1234 | 0.3376 | 1.0322 |
| Deviation $m_{0,3}$= 0 | 1-$m_{0,3}$ | 0.6351 | 1.1482 | 0.2763 | 1.0641 |
| | 1+$m_{0,3}$ | 0.6059 | 1.1286 | 0.2698 | 1.0602 |
| Variance parameters | | | | | |
| Dose $C_{0,11}$=5.427 | ¼·$C_{0,11}$ | 0.7783 | 1.2148 | 0.2975 | 0.9574 |
| | 4· $C_{0,11}$ | 0.3366 | 0.9712 | 0.3423 | 1.2434 |
| Sensitivity | ¼·$C_{0,22}$ | 0.5368 | 1.0451 | 0.3175 | 0.9496 |
| $C_{0,22}$=0.002 | 4· $C_{0,22}$ | 0.5044 | 1.1125 | 0.3299 | 1.3138 |
| Deviation $s_0/n_0$=0.09 | $s_0/4n_0$ | 0.8901 | 1.2194 | 0.3700 | 0.9245 |
| | $4s_0/n_0$ | 0.4148 | 1.0199 | 0.3639 | 2.0195 |

The model appears invariant to choices of initial values for different mean parameters, besides the increase or decrease of $m_{0,1}$ when using static dose. Increasing the deviation variance parameter by four seems to cause instability in the dynamic model and give a higher predictive error. Further, using a dynamic dose in the predictive model noticeably reduces the predictive error; hence this approach will be used in the following results.

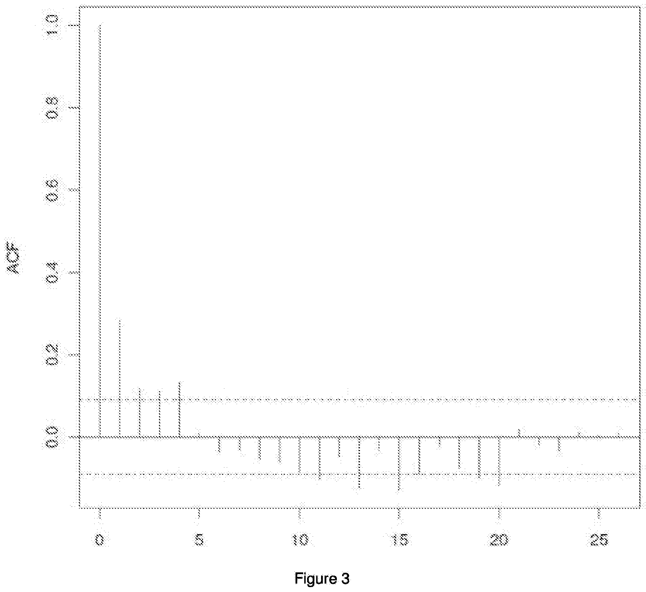

An autocorrelation analysis on residual error is performed to assess if the predictions produced by the model are mutually independent. The autocorrelation plot of residual error from lag 0 to 25 is provided in Fig. 3.

Residual correlation at lag 1 showed a small, but significant correlation. This could be due to long-term dependencies not accounted for in the predictive model.

Simulations

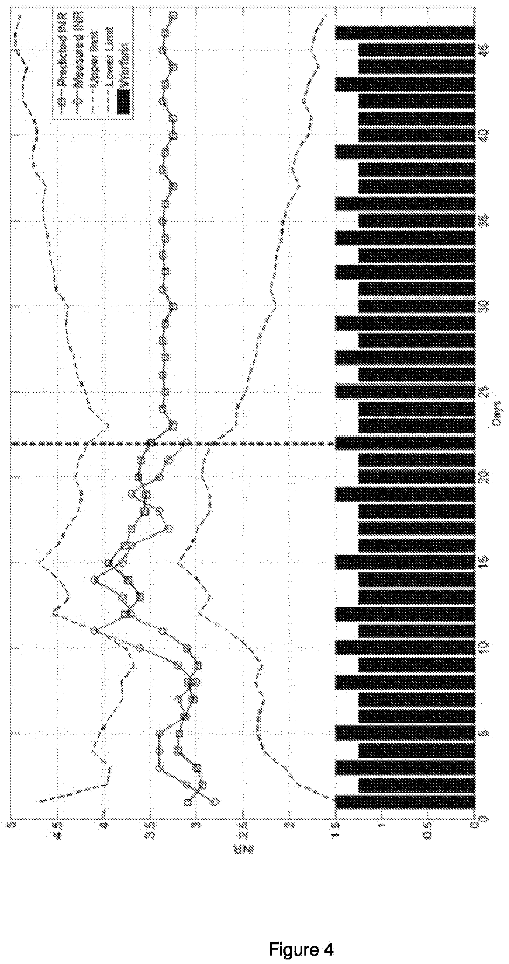

Predictions on two simulated sequences of OAT data will be carried out to evaluate the behaviour when: 1) No INR measurements, and 2) unexpected warfarin intake is provided to the model. Fig. 4 depicts the first scenario where no INR measurements are available to the model from day 22 (marked with horizontal line).

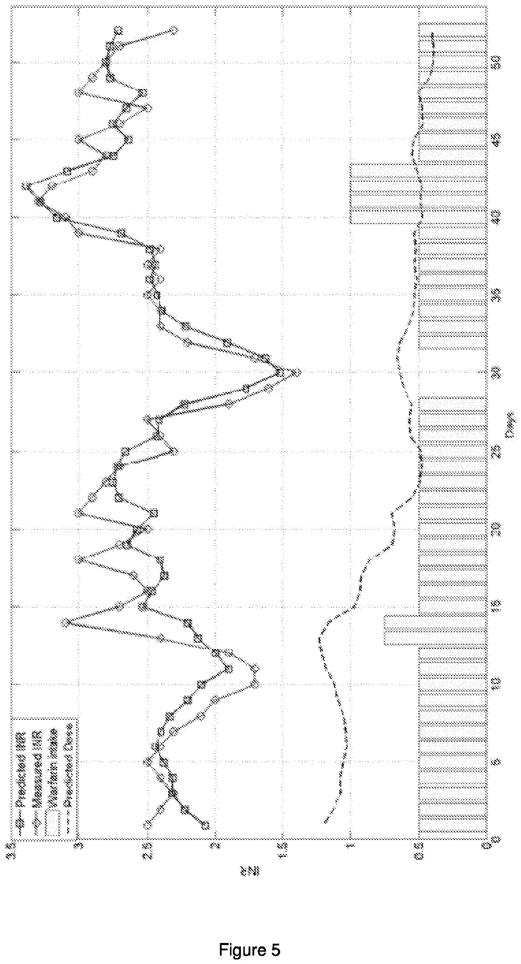

The dashed horizontal lines represent the confidence limits of INR predictions; longer time with no INR measurement increases the uncertainty of the INR prediction. An evaluation of dosage prediction is provided in Fig. 5, where a paused warfarin intake (day 28) and increased warfarin intake (day 40) is simulated.

Conclusion

Thus, in one aspect the present invention relates to the development of a predictive method and model on state-space form. The model is able to predict INR values, but also provide a theoretically correct dose of warfarin to maintain a patient on a preset target INR.

The model showed to be invariant to changes in initial population values, shown in Table 2. The example of utilizing the model on patient data, seen in Fig. 2, show how the model is able to predict the trend of INR values, most noticeably in the end of the prediction period. In addition, this patient has a target INR level at 2.5.

This target is used in the calculation of the theoretical correct dose. This is illustrated as this dose (dashed line) is above actual warfarin intake almost during the entire period. This corresponds well to the fact that this patient's INR value is below target INR during most of the prediction period.

The autocorrelation analysis resulted in an unexpected correlation at lag 1, even though the initial data analysis proved mutual uncorrelated residual error for a similar model. Reasons for this correlation could be due to the long-term effects, which have an impact on the INR values. This could be vitamin K, which has been reported to influence INR values, but also other factors as intake of medication besides warfarin, and physiological effects that have not been taking into account in the model.[21]

Inspection of confidence limits on INR predictions in Fig. 4 demonstrates the importance of providing INR measurements to the model. At the beginning of the simulated period, the model has had a limited number of INR measurements, although after five days the confidence limits seem to stabilize. At day 22 the INR measurements are not provided to the model and the Kalman filter is not updated, hence the confidence limits are expanding. However, if an INR measurement was provided in the following period (of day 22), the confidence limits of prediction would constrict. Further, the suggested dose of warfarin would remain unchanged if the sensitivity to warfarin remained unchanged during the period from day 22 to the day of a new INR measurement.

Fig. 5 reports the outcome of utilizing the model on simulated INR and warfarin to data to assess how the model behaves when presented to abnormal intake of warfarin. Of main interests is the predicted dose of warfarin depicted by the dashed line. At day 28 the warfarin intake is halted for 3 days, resulting in a drop of the INR values. However, the predicted theoretical correct dose does seem to account for such an event by not decreasing the warfarin intake, but rather slightly increasing the dose due to drop in INR levels below target value of 2.5. At day 40 the warfarin intake is doubled for four days. The dose prediction responds by a slight decrease in warfarin, as the INR levels rise above target value. The result from this analysis indicates the potential of using this predictive model as an advisory tool for either the physician or patient managing the OAT.

The model has potential to be used in different clinical settings. Patients with specific types of atrial fibrillation can in some cases benefit from direct current (DC) conversion. This type of patients is often treated with OAT. DC conversion requires an INR value at a certain level for the procedure to be safe. The developed model could be used in such a setting, where the new specific target INR is provided, and the model will predict the theoretical correct dose of warfarin to achieve that level of INR value. Anticoagulated patients who are planned for a surgical procedure requires an INR value below a certain level, e.g. 1.5, before a surgeon will perform an operation. INR should not drop too far below this value because of the risk of thrombosis. Again, the developed model could be utilized to attain this strict requirement of INR level. To further enhance the use of the model in such clinical situations, the warfarin sensitivity parameter may prove useful. During longer period of time (than data available in this study), the model will be able to depict an individual's response to warfarin changes by the sensitivity parameter. By using the model to predict INR values, physicians will have a tool that can tell how long time beforehand an adjustment is needed to be able to achieve a certain INR level.

**References**

[1] J. Ansell, J. Hirsh, E. Hylek, A. Jacobson, M. Crowther and G. Palareti, "Pharmacology and management of the vitamin K antagonists," Chest, vol. 133, pp. 160S-198S, JUN, 2008.

[2] M. C. de Assis, E. R. Rabelo, C. W. Avila, C. A. Polanczyk and L. E. Rohde, "Improved Oral Anticoagulation After a Dietary Vitamin K-Guided Strategy A Randomized Controlled Trial," Circulation, vol. 120, pp. 1115-U143, SEP 22, 2009.

[3] P. B. Nielsen, E. H. Eriksen, R. T. Milthers and O. K. Hejlesen, "Assessing importance of dietary data in anticoagulation treatment," Stud. Health Technol. Inform., vol. 150, pp. 782-786, 2009.

[4] F. R. Rosendaal, S. C. Cannegieter, F. J. M. Vandermeer and E. Briet, "A Method to Determine the Optimal Intensity of Oral Anticoagulant-Therapy," Thromb. Haemost., vol. 69, pp. 236-237, MAR 1, 1993.

[5] P. Petersen, J. Kastrup, S. Helweglarsen, G. Boysen and J. Godtfredsen, "Risk-Factors for Thromboembolic Complications in Chronic Atrial-Fibrillation - the Copenhagen Afasak Study," Arch. Intern. Med., vol. 150, pp. 819-821, APR, 1990.

[6] G. P. Samsa and D. B. Matchar, "Relationship between test frequency and outcomes of anticoagulation: A literature review and commentary with implications for the design of randomized trials of patient self-management," J. Thromb. Thrombolysis, vol. 9, pp. 283-292, APR, 2000.

[7] J. F. Lassen, I. Brandslund and S. Antonsen, "International Normalized Ratio for Prothrombin Times in Patients Taking Oral Anticoagulants - Critical Difference and Probability of Significant Change in Consecutive Measurements," Clin. Chem., vol. 41, pp. 444-447, MAR, 1995.

[8] T. D. Christensen, S. P. Johnsen, V. E. Hjortdal and J. M. Hasenkam, "Self-management of oral anticoagulant therapy: A systematic review and meta-analysis," Int. J. Cardiol., vol. 118, pp. 54-61, MAY 16, 2007.

[9] D. A. Fitzmaurice, F. D. R. Hobbs, B. C. Delaney, S. Wilson and R. Mcmanus, "Review of computerized decision support systems for oral anticoagulation management," Br. J. Haematol., vol. 102, pp. 907-909, SEP, 1998.

[10] G. Chatellier, I. Colombet and P. Degoulet, "An overview of the effect of computer-assisted management of anticoagulant therapy on the quality of anticoagulation," Int. J. Med. Inf., vol. 49, pp. 311-320, MAY, 1998.

[11] E. K. Harris, "Some Theory of Reference Values .1. Stratified (Categorized) Normal Ranges and a Method for Following an Individuals Clinical Laboratory Values," Clin. Chem., vol. 21, pp. 1457-1464, 1975.

[12] E. K. Harris, "Some Theory of Reference Values .2. Comparison of some Statistical- Models of Intraindividual Variation in Blood-Constituents," Clin. Chem., vol. 22, pp. 1343-1350, 1976.

[13] L. G. M. Jorgensen, S. G. Bottcher, E. S. Christensen, S. LundbyeChristensen and P. Winkel, "Monitoring small cell lung cancer (SCLC) by serum neuron specific enolase (S-NSE) analyses using dynamic linear models," J. Tumor Marker Oncol., vol. 11, pp. 15-21, WIN, 1996.

[14] B. R. Schlain, P. T. Lavin and C. L. Hayden, "Using Autoregressive and Random-Walk Models to Detect Trends and Shifts in Unequally Spaced Tumor Biomarker Data," Stat. Med., vol. 12, pp. 265-279, FEB, 1993.

[15] B. R. Schlain, P. T. Lavin and C. L. Hayden, "Using an Autoregressive Model to Detect Departures from Steady-States in Unequally Spaced Tumor Biomarker Data," Stat. Med., vol. 11, pp. 515-532, FEB 28, 1992.

[16] A. F. M. Smith and M. West, "Monitoring Renal-Transplants - an Application of the Multiprocess Kalman Filter," Biometrics, vol. 39, pp. 867-878, 1983.

[17] K. D. C. Stoodley and M. Mirnia, "Automatic Detection of Transients, Step Changes and Slope Changes in the Monitoring of Medical Time-Series," Statistician, vol. 28, pp. 163-170, 1979.

[18] J. Reifman, S. Rajaraman, A. Gribok and W. K. Ward, "Predictive monitoring for improved management of glucose levels," J. Diabetes Sci. Technol., vol. 1, pp. 478-486, Jul, 2007.

[19] G. Pannocchia and A. Brambilla, "Model predictive control for optimal oral anticoagulant drug administration," AICHE J., vol. 52, pp. 3315-3320, 2006.

[20] P. Brønnum Nielsen, S. Lundbye-Christensen, T. Bjerregaard Larsen, L. Hvilsted Rasmussen, S. Risom Kristensen, A. Münster and O. K. Hejlesen, "Data Mining to Assess Variations in Oral Anticoagulant Treatment," 2010.

[21] R. Couris, G. Tataronis, W. McCloskey, L. Oertel, G. Dallal, J. Dwyer and J. B. Blumberg, "Dietary vitamin K variability affects International Normalized Ratio (INR) coagulation indices," Int. J. Vitam. Nutr. Res., vol. 76, pp. 65-74, MAR, 2006.

**Claims**

1. A computer implemented method for predicting an International Normalized Ratio for a patient receiving an anti-coagulation treatment by determining an INR value, comprising the steps of

   a) inputting to a processor the following values:

   - a target response T of the INR value,
   - parameters a1 and a2 describing the individual sensitivity towards dosage variation of an anticoagulant administered to the patient,
   - values of the administered dose of the anticoagulant dt-1 and dt-2 administered at time t-1 and t-2,
   - a theoretical dose D of the anticoagulant to administer for obtaining the target response T, and
   - $\varepsilon_t$ wherein $\varepsilon_t$ describes an autoregressive process given by $\varepsilon_t = \rho \cdot \varepsilon_{t-1} + v_t$ wherein p is a constant quantifying the day-to-day correlation between $\varepsilon_t$ and $\varepsilon_{t-1}$, and wherein $v_t$ are mutually independent, normally distributed with parameters $N(0, \sigma^2)$, and

   b) computing the INRt value by inputting said values into the equation:

$$INR_t = T + a_1(d_{t-1} - D) + a_2(d_{t-2} - D) + \varepsilon_t \qquad (ii)$$

2. The method according to claim 1, wherein $\varepsilon_t$ is a first order autoregressive process (AR(1)).

3. The method according to any of claims 1-2, wherein the anticoagulant is a vitamin K antagonist.

4. The method according to any of claims 1-3, wherein the anticoagulant is warfarin.

5. The method according to any of the preceding claims, wherein said individual is a patient at increased risk of thromboembolism.

6. The method according to any of the preceding claims, wherein said individual is a patient suffering from any one or more diseases/conditions selected from the group of diseases/conditions consisting of atrial fibrillation, heart valve replacement, deep venous thrombosis and pulmonary embolism.

7. The method according to any of the preceding claims, wherein the target response "T" is determined.

8. The method according to any of the preceding claims, wherein the theoretical dose "D" is determined.

9. A calculator for predicting an International Normalized Ratio for a patient receiving an anti-coagulation treatment by determining an INRt value, comprising an input module for inputting one or more values, a computation module for performing one or more calculations, and an output module for displaying one or more results, wherein the calculator is configured to determine the INRt value by means of the steps:

a) inputting the following values to the input module:

- a target response T of the INR value,
- parameters a1 and a2 describing the individual sensitivity towards dosage variation of an anticoagulant administered to the patient,
- values of the administered dose of the anticoagulant dt-1 and dt-2 administered at time t-1 and t-2,
- a theoretical dose D of the anticoagulant to administer for obtaining the target response T, and
- $\varepsilon_t$, wherein $\varepsilon_t$ describes an autoregressve process given by $\varepsilon_t = \rho \cdot \varepsilon_{t-1} + v_t$ wherein $\rho$ is a constant quantifying the day-to-day correlation between $\varepsilon_t$ and $\varepsilon_{t-1}$, and wherein $v_t$ are mutually independent, normally distributed with parameters $N(0, \sigma^2)$,

b) in the computation module, computing the INRt value by inputting said values into the equation:

$$INR_t = T + a_1(d_{t-1} - D) + a_2(d_{t-2} - D) + \varepsilon_t \qquad (ii) \quad ,$$

c) outputting the result to the output module; and
d) displaying the result on the output module.

**Patentansprüche**

1. Computerimplementiertes Verfahren zur Vorhersage einer International Normalized Ratio für einen Patienten, der eine Antikoagulationsbehandlung erhält, durch Bestimmen eines INR-Werts, umfassend die Schritte von

a) Eingeben der folgenden Werte in einen Prozessor:

- eine Zielantwort T des INR-Werts,
- Parameter a1 und a2, die die individuelle Empfindlichkeit gegenüber Dosisschwankungen eines Antikoagulans beschreiben, das dem Patienten verabreicht wird,
- Werte der verabreichten Dosis des Antikoagulans dt-1 und dt-2, das zum Zeitpunkt t-1 und t-2 verabreicht wird,
- eine theoretische Dosis D des zu verabreichenden Antikoagulans, um die Zielantwort T zu erhalten, und
- $\varepsilon_t$, wobei $\varepsilon_t$ einen autoregressiven Prozess gegeben durch $\varepsilon_t = \rho \cdot \cdot \varepsilon_{t-1} + v_t$ beschreibt, wobei p eine Konstante ist, die die tägliche Korrelation zwischen $\varepsilon_t$ und $\varepsilon_{t-1}$ quantifiziert und wobei $v_t$ voneinander unab-hängig, normalverteilt mit den Parametern $N(0, \sigma^2)$ sind, und

b) Berechnen des INRt-Werts durch Eingeben der Werte in die Gleichung:

$$INR_t = T + a_1(d_{t-1} - D) + a_2(d_{t-2} - D) + \varepsilon_t \qquad (ii) \quad .$$

2. Verfahren nach Anspruch 1, wobei $\varepsilon_t$ ein autoregressiver Prozess erster Ordnung (AR(1)) ist.

3. Verfahren nach einem der Ansprüche 1-2, wobei das Antikoagulans ein Vitamin-K-Antagonist ist.

4. Verfahren nach einem der Ansprüche 1-3, wobei das Antikoagulans Warfarin ist.

5. Verfahren nach einem der vorstehenden Ansprüche, wobei die Person ein Patient ist, bei dem ein erhöhtes Risiko einer Thromboembolie besteht.

6. Verfahren nach einem der vorstehenden Ansprüche, wobei die Person ein Patient ist, der an einer oder mehreren Erkrankungen/Zuständen leidet, die ausgewählt sind aus der Gruppe von Erkrankungen/Zuständen, bestehend aus aus Vorhofflimmern, Herzklappenersatz, tiefer Venenthrombose und Lungenembolie.

**7.** Verfahren nach einem der vorstehenden Ansprüche, wobei die Zielantwort "T" bestimmt wird.

**8.** Verfahren nach einem der vorstehenden Ansprüche, wobei die theoretische Dosis "D" bestimmt wird.

**9.** Rechner zum Vorhersagen einer International Normalized Ratio für einen Patienten, der eine Antikoagulationsbehandlung erhält, durch Bestimmen eines INRt-Werts, umfassend ein Eingabemodul zum Eingeben eines oder mehrerer Werte, ein Berechnungsmodul zum Durchführen einer oder mehrerer Berechnungen und ein Ausgabemodul zum Anzeigen eines oder mehrerer Ergebnisse, wobei der Rechner konfiguriert ist, um den INRt-Wert mittels der Schritte zu bestimmen:

   a) Eingeben der folgenden Werte in das Eingabemodul:

   - eine Zielantwort T des INR-Werts,
   - Parameter a1 und a2, die die individuelle Empfindlichkeit gegenüber Dosisschwankungen eines Antikoagulans beschreiben, das dem Patienten verabreicht wird,
   - Werte der verabreichten Dosis des Antikoagulans dt-1 und dt-2, das zum Zeitpunkt t-1 und t-2 verabreicht wird,
   - eine theoretische Dosis D des zu verabreichenden Antikoagulans, um die Zielantwort T zu erhalten, und
   - $\varepsilon_t$, wobei $\varepsilon_t$ einen autoregressiven Prozess gegeben durch $\varepsilon_t = \rho \cdot \varepsilon_{t-1} + v_t$ beschreibt, wobei $\rho$ eine Konstante ist, die die tägliche Korrelation zwischen $\varepsilon_t$ und $\varepsilon_{t-1}$ quantifiziert und wobei $v_t$ voneinander unabhängig, normalverteilt mit den Parametern $N(0, \sigma^2)$ sind,

   b) Berechnen des INRt-Werts in dem Berechnungsmodul durch Eingeben der Werte in die Gleichung:

$$INR_t = T + a_1(d_{t-1} - D) + a_2(d_{t-2} - D) + \varepsilon_t \qquad (ii)$$,

   c) Ausgeben des Ergebnisses an das Ausgabemodul; und
   d) Anzeigen des Ergebnisses auf dem Ausgabemodul.


**Revendications**

**1.** Procédé implémenté par ordinateur pour prédire un rapport international normalisé (INR International Normalized Ratio) pour un patient recevant un traitement anticoagulant en déterminant une valeur INR, comprenant les étapes consistant à :

   a) entrer dans un processeur les valeurs suivantes :

   - une réponse cible T de la valeur INR,
   - les paramètres a1 et a2 décrivant la sensibilité individuelle vis-à-vis de la variation du dosage d'un anticoagulant administré au patient,
   - les valeurs de la dose administrée des anticoagulants dt-1 et dt-2 administrés aux temps t-1 et t-2,
   - une dose théorique D de l'anticoagulant à administrer pour obtenir la réponse cible T et
   - $\varepsilon_t$ dans lequel $\varepsilon_t$ décrit un processus autorégressif donné par $\varepsilon_t = \rho \cdot \varepsilon_{t-1} + v_t$ où $\rho$ est une constante quantifiant la corrélation au jour le jour entre $\varepsilon_t$ et $\varepsilon_{t-1}$ et dans lequel $v_t$ sont mutuellement indépendants, distribués normalement avec les paramètres $N(0, \sigma^2)$ et

   b) calculer la valeur INRt en introduisant lesdites valeurs dans l'équation :

$$INR_t = T + a_1(d_{t-1} - D) + a_2(d_{t-2} - D) + \varepsilon_t \qquad (ii)$$.

**2.** Procédé selon la revendication 1, dans lequel $\varepsilon_t$ est un processus autorégressif de premier ordre (AR(1)).

**3.** Procédé selon l'une quelconque des revendications 1 et 2, dans lequel l'anticoagulant est un antagoniste de la vitamine K.

**4.** Procédé selon l'une quelconque des revendications 1 à 3, dans lequel l'anticoagulant est la warfarine.

**5.** Procédé selon l'une quelconque des revendications précédentes, dans lequel ledit individu est un patient présentant un risque accru de thrombo-embolie.

**6.** Procédé selon l'une quelconque des revendications précédentes, dans lequel ledit individu est un patient souffrant d'une ou plusieurs maladies/affections sélectionnées dans le groupe des maladies/affections comprenant la fibrillation auriculaire, le remplacement valvulaire cardiaque, la thrombose veineuse profonde et l'embolie pulmonaire.

**7.** Procédé selon l'une quelconque des revendications précédentes, dans lequel la réponse cible « T » est déterminée.

**8.** Procédé selon l'une quelconque des revendications précédentes, dans lequel la dose théorique « D » est déterminée.

**9.** Calculateur pour prédire un INR pour un patient recevant un traitement anticoagulant en déterminant une valeur INRt, comprenant un module d'entrée pour entrer une ou plusieurs valeurs, un module de calcul pour effectuer un ou plusieurs calculs et un module de sortie pour afficher un ou plusieurs résultats, dans lequel le calculateur est configuré pour déterminer la valeur INRt au moyen des étapes suivantes :

a) entrer les valeurs suivantes dans le module d'entrée :

- une réponse cible T de la valeur INR,
- les paramètres a1 et a2 décrivant la sensibilité individuelle vis-à-vis de la variation du dosage d'un anticoagulant administré au patient,
- les valeurs de la dose administrée des anticoagulants $d_{t-1}$ et $d_{t-2}$ administrés aux temps t-1 et t-2,
- une dose théorique D de l'anticoagulant à administrer pour obtenir la réponse cible T et
- $\varepsilon_t$, dans lequel $\varepsilon_t$ décrit un processus autorégressif donné par $\varepsilon_t = \rho \cdot \varepsilon_{t-1} + v_t$ où p est une constante quantifiant la corrélation au jour le jour entre $\varepsilon_t$ et $\varepsilon_{t-1}$ et dans lequel $v_t$ sont mutuellement indépendants, distribués normalement avec les paramètres $N(0, \sigma^2)$,

b) dans le module de calcul, calculer la valeur INRt en introduisant lesdites valeurs dans l'équation :

$$INR_t = T + a_1(d_{t-1} - D) + a_2(d_{t-2} - D) + \varepsilon_t \qquad (ii) \qquad ,$$

c) envoyer le résultat au module de sortie ; et
d) afficher le résultat sur le module de sortie.

Figure 1

Figure 2

Figure 3

Figure 4

Figure 5

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **J. ANSELL ; J. HIRSH ; E. HYLEK ; A. JACOBSON ; M. CROWTHER ; G. PALARETI.** Pharmacology and management of the vitamin K antagonists. *Chest,* June 2008, vol. 133, 160S-198S
- **M. C. DE ASSIS ; E. R. RABELO ; C. W. AVILA ; C. A. POLANCZYK ; L. E. ROHDE.** Improved Oral Anticoagulation After a Dietary Vitamin K-Guided Strategy A Randomized Controlled Trial. *Circulation,* 22 September 2009, vol. 120, 1115-U143
- **P. B. NIELSEN ; E. H. ERIKSEN ; R. T. MILTHERS ; O. K. HEJLESEN.** Assessing importance of dietary data in anticoagulation treatment. *Stud. Health Technol. Inform.,* 2009, vol. 150, 782-786
- **F. R. ROSENDAAL ; S. C. CANNEGIETER ; F. J. M. VANDERMEER ; E. BRIET.** A Method to Determine the Optimal Intensity of Oral Anticoagulant-Therapy. *Thromb. Haemost.,* 01 March 1993, vol. 69, 236-237
- **P. PETERSEN ; J. KASTRUP ; S. HELWEGLARSEN ; G. BOYSEN ; J. GODTFRED-SEN.** Risk-Factors for Thromboembolic Complications in Chronic Atrial-Fibrillation - the Copenhagen Afasak Study. *Arch. Intern. Med.,* April 1990, vol. 150, 819-821
- **G. P. SAMSA ; D. B. MATCHAR.** Relationship between test frequency and outcomes of anticoagulation: A literature review and commentary with implications for the design of randomized trials of patient self-management. *J. Thromb. Thrombolysis,* April 2000, vol. 9, 283-292
- **J. F. LASSEN ; I. BRANDSLUND ; S. ANTONSEN.** International Normalized Ratio for Prothrombin Times in Patients Taking Oral Anticoagulants - Critical Difference and Probability of Significant Change in Consecutive Measurements. *Clin. Chem.,* March 1995, vol. 41, 444-447
- **T. D. CHRISTENSEN ; S. P. JOHNSEN ; V. E. HJORTDAL ; J. M. HASENKAM.** Self-management of oral anticoagulant therapy: A systematic review and meta-analysis. *Int. J. Cardiol.,* 16 May 2007, vol. 118, 54-61
- **D. A. FITZMAURICE ; F. D. R. HOBBS ; B. C. DELANEY ; S. WILSON ; R. MCMANUS.** Review of computerized decision support systems for oral anticoagulation management. *Br. J. Haematol.,* September 1998, vol. 102, 907-909

- **G. CHATELLIER ; I. COLOMBET ; P. DEGOULET.** An overview of the effect of computer-assisted management of anticoagulant therapy on the quality of anticoagulation. *Int. J. Med. Inf.,* May 1998, vol. 49, 311-320
- **E. K. HARRIS.** Some Theory of Reference Values .1. Stratified (Categorized) Normal Ranges and a Method for Following an Individuals Clinical Laboratory Values. *Clin. Chem.,* 1975, vol. 21, 1457-1464
- **E. K. HARRIS.** Some Theory of Reference Values .2. Comparison of some Statistical- Models of Intraindividual Variation in Blood-Constituents. *Clin. Chem.,* 1976, vol. 22, 1343-1350
- **L. G. M. JORGENSEN ; S. G. BOTTCHER ; E. S. CHRISTENSEN ; S. LUNDBYECHRISTENSEN ; P. WINKEL.** Monitoring small cell lung cancer (SCLC) by serum neuron specific enolase (S-NSE) analyses using dynamic linear models. *J. Tumor Marker Oncol.,* 1996, vol. 11, 15-21
- **B. R. SCHLAIN ; P. T. LAVIN ; C. L. HAYDEN.** Using Autoregressive and Random-Walk Models to Detect Trends and Shifts in Unequally Spaced Tumor Biomarker Data. *Stat. Med.,* February 1993, vol. 12, 265-279
- **B. R. SCHLAIN ; P. T. LAVIN ; C. L. HAYDEN.** Using an Autoregressive Model to Detect Departures from Steady-States in Unequally Spaced Tumor Biomarker Data. *Stat. Med.,* 28 February 1992, vol. 11, 515-532
- **A. F. M. SMITH ; M. WEST.** Monitoring Renal-Transplants - an Application of the Multiprocess Kalman Filter. *Biometrics,* 1983, vol. 39, 867-878
- **K. D. C. STOODLEY ; M. MIRNIA.** Automatic Detection of Transients, Step Changes and Slope Changes in the Monitoring of Medical Time-Series. *Statistician,* 1979, vol. 28, 163-170
- **J. REIFMAN ; S. RAJARAMAN ; A. GRIBOK ; W. K. WARD.** Predictive monitoring for improved management of glucose levels. *J. Diabetes Sci. Technol.,* July 2007, vol. 1, 478-486
- **G. PANNOCCHIA ; A. BRAMBILLA.** Model predictive control for optimal oral anticoagulant drug administration. *AICHE J.,* 2006, vol. 52, 3315-3320
- **P. BRØNNUM NIELSEN ; S. LUNDBYE-CHRISTENSEN ; T. BJERREGAARD LARSEN ; L. HVILSTED RASMUSSEN ; S. RISOM KRISTENSEN ; A. MÜNSTER ; O. K. HEJLESEN.** *Data Mining to Assess Variations in Oral Anticoagulant Treatment,* 2010

- **R. COURIS ; G. TATARONIS ; W. MCCLOSKEY ; L. OERTEL ; G. DALLAL ; J. DWYER ; J. B. BLUMBERG.** Dietary vitamin K variability affects International Normalized Ratio (INR) coagulation indices. *Int. J. Vitam. Nutr. Res.,* March 2006, vol. 76, 65-74